# FASCICULE DE BREVET EUROPEEN

(11) **EP 0 929 525 B1**
(45) Date de publication et mention de la délivrance du brevet: **04.09.2002**
(21) Numéro de dépôt: 97943000.6
(22) Date de dépôt: 26.09.1997
(51) Int. Cl.: C07D 215/00

(54) **HETEROARYLOXYETHYLAMINES COMME LIGANDS DU RECEPTEUR 5-HT1A**
HETEROARYLOXYETHYLAMINEN ALS 5-HT1A REZEPTOR LIGANDEN
HETEROARYLOXYETHYLAMINES AS 5-HT1A RECEPTOR LIGANDS

(30) Priorité: 27.09.1996 FR 9611798
(43) Date de publication de la demande: 21.07.1999
(73) Titulaire: SOCIETE DE CONSEILS DE RECHERCHES ET D'APPLICATIONS SCIENTIFIQUES (S.C.R.A.S.), 75016 Paris (FR)
(72) Inventeur: BIGG, Denis, F-91190 Gif-sur-Yvette (FR); GALCERA, Marie-Odile, F-91070 Bondoufle (FR)
(74) Mandataire: Bourgouin, André
(86) Numéro de dépôt international: FR9701691
(87) Numéro de publication internationale: WO98013349

(56) Documents cités:
- EP-A- 0 512 755
- WO-A-92/03426
- WO-A-96/31461
- ZDISLAW CHILMONCZYK ET AL: "Buspirone analogues as ligands of the 5-HT1A receptor. 1. ..." JOURNAL OF MEDICINAL CHEMISTRY, vol. 38, no. 10, - 12 mai 1995 WASHINGTON US, pages 1701-1710, XP002032675
- MARCEL F. HIBERT ET AL: "Graphics computer-aided receptor mapping as ..." JOURNAL OF MEDICINAL CHEMISTRY, vol. 31, no. 6, - 1988 WASHINGTON US, pages 1087-1093, XP002032676

## Description

Les ligands 5-HT_{1A} peuvent être utiles pour le traitement de l'anxiété, la dépression et l'hypertension (Brain 5-HT_{1A} Receptors : Behavioural and Neurochemical Pharmacology ; Editors C.T. Dourish, S. Ahlenius, P.H. Huston ; Ellis Horwood LTD, Chischester (1987)).

Il a été également démontré que les ligands 5-HT_{1A} inhibent la sécrétion de l'acide gastrique (D.C. Evans, J.S. Gidda, Gastroenterology, 104, A76 (1993)), montrent des effets anti-émétiques (F. Okada, Y. Torii, H. Saito, N. Matsuki, Jpn J. Pharmacol., 64, 109(1994)) et agissent sur la motilité du système gastrointestinal (Serotonin and Gastrointestinal Function, Editors T.S. Gaginella, J.J. Galligan ; CRC Press, Boca Raton (1995)).

La présente invention concerne de nouvelles hétéroaryloxyéthylamines possédant une haute affinité pour le récepteur 5-HT_{1A}, un procédé pour leur préparation, des compositions pharmaceutiques les contenant et leur utilisation notamment en tant qu'inhibiteurs de sécrétion d'acide gastrique ou en tant qu'anti-émétiques.

L'invention a ainsi pour objet les produits de formule générale I 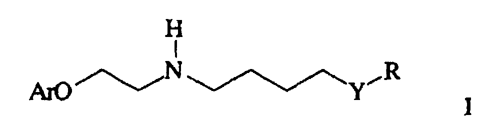 dans laquelle
- Ar: représente un hétéroaryle bicyclique, éventuellement substitué par un ou plusieurs substituants, tel que défini ci-dessous;
- Y: représente le radical -C(O)NH- ou -NHC(O)- ;
- R: représente un radical alkyle inférieur; un cycloalkyle éventuellement substitué par un ou plusieurs alkyle(s) inférieur(s) ; ou un phényle éventuellement substitué par un ou plusieurs substituants choisis parmi les radicaux hydroxy, cyano, halo, trifluorométhyle, alkyle inférieur, alkoxy inférieur ;
ainsi que les sels de ces produits.

L'invention est particulièrement caractérisée en ce que Ar représente un radical hétéroaryle bicyclique contenant un ou deux atomes d'azote, et le ou les substituant(s) que peut porter le radical que représente Ar sont choisis parmi les radicaux hydroxy, nitro, halo, alkyle inférieur, alkoxy inférieur, alkyle inférieur carbonyle.

Dans les définitions indiquées ci-dessus, l'expression halo représente fluoro, chloro, bromo ou iodo et de préférence chloro. L'expression alkyle inférieur représente un radical alkyle ayant de 1 à 6 atomes de carbone, linéaire ou ramifié, tels que les radicaux méthyle, éthyle, propyle, isopropyle, butyle, isobutyle, sec-butyle, tert-butyle, isopentyle, néopentyle et hexyle.

Les radicaux cycloalkyles peuvent être choisis parmi les radicaux monocycliques saturés ayant de 3 à 7 atomes de carbone tels que les radicaux cyclopropyle, cyclobutyle, cyclopentyle, cyclohexyle et cycloheptyle.

Les radicaux alkoxy inférieurs correspondent aux radicaux alkyle inférieurs indiqués ci-dessus. On préfère les radicaux méthoxy, éthoxy ou isopropyloxy.

Les radicaux hétéroaryles bicycliques contenant un ou deux atomes d'azote peuvent être choisis parmi les radicaux corespondant aux composés indole, indazole, isoquinoléine, quinoléine, quinoxaline et quinazoline.

Les produits de formule I peuvent former des sels d'addition avec les acides, en particulier les acides pharmacologiquement acceptables.

Des exemples de sels sont donnés ci-après dans la partie expérimentale.

L'invention a plus particulièrement pour objet les produits de formule générale 1 telle que définie ci-dessus, caractérisée en ce que Ar représente le radical quinolyle ou quinoxalinyle, et le ou les substituant(s) que peut porter le radical que représente Ar sont choisis parmi les radicaux hydroxy, chloro, méthyle.

Plus particulièrement, l'invention a pour objet les produits décrits ci-après dans les exemples, en particulier les produits répondant aux formules suivantes :
- N-[4-({2-(8-quinolyloxy)éthyl}amino)butyl]*tert*butanamide,
- N-[4-({2-(8-quinolyloxy)éthyl}amino)butyl]néopentanamide,
- N-[4-({2-(8-quinolyloxy)éthyl}amino)butyl]cyclohexanamide,
- 4-méthyl-N-[4-({2-(8-quinolyloxy)éthyl}amino)butyl]cyclohexanamide,
- N-[4-({2-(8-quinolyloxy)éthyl}amino)butyl]benzamide,
- N-[4-({2-(8-(5-chloro)quinolyloxy)éthyl}amino)butyl]néopentanamide,
- N-[4-({2-(8-(2-méthyl)quinolyloxy)éthyl}amino)butyl]néopentanamide,
- N-[4-({2-(5-quinolyloxy)éthyl}amino)butyl]benzamide,
- N-[4-({2-(5-quinoxalyloxy)éthyl}amino)butyl]benzamide,
- N-[4-({2-(8-(2-hydroxy)quinolyloxy)éthyl}amino)butyl]benzamide,
- N-cyclohexyl-5-[(2-{8-quinolyloxy}éthyl)amino]pentanamide,
- N-néopentyl-5-[(2-{8-quinolyloxy}éthyl)amino]pentanamide,
- N-néopentyl-5-[(2-{5-quinoxalyloxy}éthyl)amino]pentanamide,
ainsi que les sels de ces composés avec les acides minéraux ou organiques.

L'invention a également pour objet un procédé de préparation des produits de formule générale I telle que définie ci-dessus, caractérisé en ce que

l'on fait réagir un produit de formule II 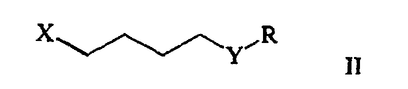 dans laquelle Y et R ont la signification indiquée ci-dessus et X représente un halogène ou un pseudo halogène, avec la N-benzyléthanolamine de formule 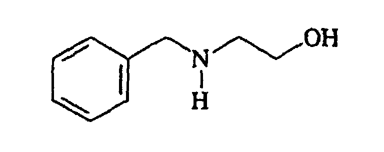 pour obtenir un produit de formule III 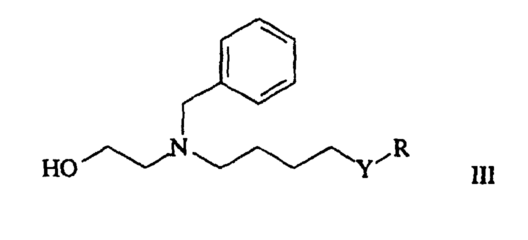 que l'on transforme en produit de formule IV 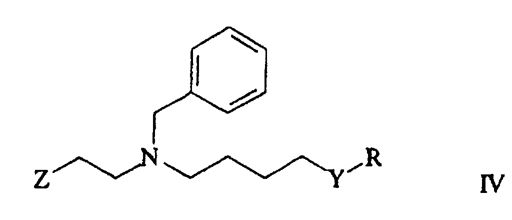 dans laquelle Z représente un halogène ou un pseudo halogène, produit de formule IV que l'on transforme en produit de formule V 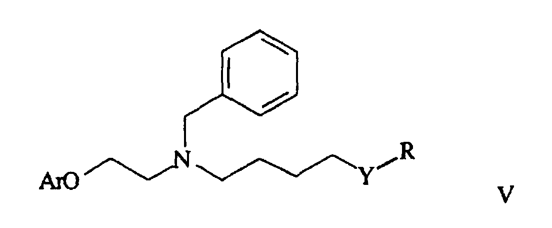 dans laquelle Ar a la signification indiquée ci-dessus, produit de formule V dont la fonction benzyle est clivée pour obtenir le produit de formule I, et produit de formule 1 que l'on peut transformer en sels d'acide par action de l'acide correspondant.

Dans les synthèses telles que présentées ci-dessus, X et Z représentent, indépendamment, un groupe partant tel que chloro, bromo, iodo, méthanesulphonyloxy, benzènesulphonyloxy ou p-toluènesulphonyloxy, en d'autres termes, un groupement halogène ou pseudo halogène.

Des composés de formule générale III peuvent être préparés en chauffant simplement un composé de formule générale II avec un excès de N-benzyléthanolamine en l'absence de solvant, de préférence sous atmosphère d'azote et à une température comprise entre 70°C et 85°C. Alternativement, les composés de formule générale III peuvent être préparés en chauffant entre 60°C et 70°C un composé de formule générale II avec la N-benzyléthanolamine dans un solvant polaire tel que le diméthylformamide, en présence d'un capteur d'acide tel que le carbonate de potassium, et éventuellement en présence d'un catalyseur tel que l'iodure de potassium.

Les composés de formule générale III ainsi obtenus peuvent être transformés, par exemple, en chlorures de formule générale IV (Z = Cl) par réaction avec le chlorure de méthanesulphonyle dans un solvant inerte tel que le dichlorométhane, en présence d'une base organique telle que la triéthylamine à température ambiante.

Des composés de formule générale V peuvent être préparés à partir de composés de formule générale IV en faisant réagir ces derniers avec un anion aryloxyde produit à partir du dérivé hydroxyaryle approprié de formule ArOH, en utilisant soit une base telle que l'hydrure de sodium dans un solvant aprotique dipolaire tel que le diméthylformamide, soit en chauffant à 80°C des composés de formule générale IV dans un solvant aprotique dipolaire tel que le diméthylformamide en présence d'une base, par exemple, une base inorganique, de préférence le carbonate de potassium.

Les composés de formule générale I sont obtenus en déprotégeant les composés de formule générale V selon les méthodes générales connues pour la N-débenzylation, par exemple, la réaction avec un chloroformate tel que l'α-chloroéthylchloroformate suivie d'une méthanolyse, ou l'hydrogénation catalytique dans la mesure où celle-ci est compatible avec les substituants sur le(s) noyau(x) aryle(s) des composés de formule générale V. D'autres méthodes de débenzylation, telles que décrites dans "Protective Groups in Organic Synthesis" [T.W. Green, P.G.M. Wuts ; 2nd Édition, J. Wiley and Sons Inc., p. 364-6 (1991)] peuvent également être utilisées.

La salification éventuelle des produits de formule I est également réalisée selon les méthodes usuelles, par exemple, celles indiquées ci-après dans la partie expérimentale.

Les composés de la présente invention possèdent d'intéressantes propriétés pharmacologiques. C'est ainsi que l'on a découvert que les composés de la présente invention ont une haute affinité pour le récepteur 5HT_{1A}. Les composés de la présente invention peuvent ainsi être utilisés dans différentes applications thérapeutiques.

Les composés peuvent inhiber la sécrétion de l'acide gastrique. Ils peuvent inhiber le vomissement induit, par exemple, par le cis-platine. Ainsi, les composés de l'invention peuvent être utilisés en tant qu'anti-émétiques ou pour le traitement des maladies dans lesquelles il est nécessaire, ou souhaitable, de réduire la sécrétion de l'acide gastrique, par exemple, les ulcères gastriques ou duodénaux, les gastrites, le reflux gastroesophagial, la dyspepsie gastrique, le syndrome de Zollinger-Ellison, les nausées.

Les composés de l'invention peuvent également présenter une activité sur la vidange gastrique et la motilité intestinale. Ils peuvent ainsi être utilisés pour lutter contre la constipation, l'atonie post-chirurgicale, la gastroparésie.

Ils peuvent également être utilisés pour lutter contre certaines maladies du système nerveux telles que l'anxiété, la dépression, les troubles du sommeil tels que l'insomnie, la dépendance vis à vis de certaines drogues, la maladie d'Alzheimer, le vertige, les maladies de l'alimentation telles que l'anorexie. Les composés de l'invention peuvent également être utilisés pour traiter des maladies du système cardiovasculaire, notamment l'hypertension.

On trouvera ci-après, dans la partie expérimentale, une illustration des propriétés pharmacologiques des composés de l'invention.

Ces propriétés rendent les produits de formule I aptes à une utilisation pharmaceutique. La présente demande a également pour objet, à titre de médicaments, les produits de formule I telle que définie ci-dessus, ainsi que les sels d'addition avec les acides minéraux ou organiques pharmaceutiquement acceptables desdits produits de formule I, ainsi que les compositions pharmaceutiques contenant, à titre de principe actif, l'un au moins des médicaments tels que définis ci-dessus.

L'invention concerne aussi des compositions pharmaceutiques contenant un composé de l'invention ou un sel additif d'acide pharmaceutiquement acceptable de celui-ci, en association avec un support pharmaceutiquement acceptable. La composition pharmaceutique peut être sous forme d'un solide, par exemple, des poudres, des granules, des comprimés, des gélules ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau, additionnés à des huiles ou des graisses pharmaceutiquement acceptables. Les compositions liquides stériles peuvent être utilisées pour les injections intramusculaires, intrapéritonéales ou sous-cutanées et les compositions stériles peuvent également être administrée par voie intraveineuse.

L'invention a également pour objet l'utilisation des produits de formule I telle que définie ci-dessus, pour la préparation de médicaments antiémétiques, de médicaments destinés à réduire la sécrétion gastrique, de médicaments destinés à accélérer la vidange gastrique, de médicaments destinés à modifier le transit intestinal, de médicaments destinés à traiter l'anxiété, la dépression, les troubles du sommeil ainsi que de médicaments destinés à traiter les maladies cardiovasculaires.

L'invention a également pour objet, à titre de produits industriels nouveaux, et notamment à titre de produits industriels nouveaux destinés à la préparation des produits de formule I, les produits de formule V telle que décrite ci-dessus.

Les produits de départ de l'invention sont des produits connus ou qui peuvent être préparés à partir de produits connus selon des méthodes classiques connues de l'homme de l'art. On peut citer les références suivantes : la N-benzyléthanolamine est un produit commercialisé par exemple par la firme ACROS.

Dans le cas où Y = NHC(O)-, les produits de formule II peuvent être préparés à partir de l'ω-hydroxy-butylamide selon les méthodes usuelles de synthèse des halogénures ou pseudo-halogénures : par exemple, dans le cas où X est un radical mésyle, ces composés II peuvent être préparés selon la méthode décrite dans J. Org. Chem., 35(9), 3195-6 (1970). L'ω-hydroxy-butylamide est lui-même obtenu par condensation de l'acide carboxylique correspondant ou de ses dérivés tels que le chlorure d'acide sur le 4-amino-1-butanol (produit commercialisé par exemple par la firme ACROS) selon des méthodes connues.

Dans le cas où Y = -C(O)NH-, les produits de formule II peuvent être obtenus par action de l'amine correspondante, de formule RNH₂, sur un acide pentanoïque possédant un groupement halogène ou pseudo-halogène en position 5, un atome de brome par exemple ou un dérivé activé de cet acide tel que le chlorure d'acide par exemple.

Les composés de formule ArOH sont des produits commerciaux ou peuvent être préparés selon des procédés décrits dans la littérature (J.Org.Chem., 33(3), 1089-92 (1968) ; Helv. Chim. Acta 34(2), 427-430 (1951).

Les exemples suivants sont présentés pour illustrer les procédures ci-dessus et ne doivent en aucun cas être considérés comme une limite à la portée de l'invention.

### PARTIE EXPERIMENTALE

### EXEMPLE 1

### N-[4-({2-(8-quinolyloxy)éthyl}amino)butyl] benzamide

(I, Ar = 8-quinolyle, R = phényle, Y = -NHC(O)- : composé n°5, Tableau 1)

### 1ère étape

### N-[4-(benzyl-(2-hydroxyéthyl)amino)butyl]benzamide

### (III, R = phényle, Y=-NHC(O)-)

On chauffe un mélange de N-benzyléthanolamine (60,5 g, 56,8 ml, 0,4 mole) et de N-[4-(méthanesulphonyloxy)butyl]benzamide (51,5 g, 0,19 mole) pendant 2 heures à 75-80°C sous atmosphère d'azote. Le mélange réactionnel est refroidi, repris dans du dichlorométhane (400 ml), la solution est lavée à l'eau (3 x 100 ml), puis séchée sur du sulfate de magnésium. La solution est filtrée et le solvant éliminé sous pression réduite pour obtenir une huile qui cristallise avec le diisopropyléther. On obtient 51,5 g (83%) du composé souhaité, sous forme de cristaux blancs, pf = 61-62°C.

### 2ème étape

### N-[4-(benzyl-(2-chloroéthyl)amino)butyl]benzamide

### (IV, R=phényle, Y=-NHC(O)-)

On ajoute, goutte à goutte, du chlorure de méthanesulphonyle (14,8 g, 10 ml, 0,13 mole), sous agitation, à une solution refroidie (5°C) de N-[4{benzyl (2-hydroxyéthyl)amino}butyl] benzamide (35 g, 0,11 mole) dans du dichlorométhane (300 ml). La solution est ramenée à température ambiante et, après 1,5 heure, on ajoute de la triéthylamine (13 g, 18 ml, 0,13 mole) et on maintient l'agitation pendant encore 1,5 heure. Le mélange réactionnel est lavé à l'eau glacée (150 ml), à l'eau salée saturée (2 x 100 ml) et séché sur du sulfate de magnésium. La filtration et l'évaporation du solvant sous pression réduite donnent 36,6 g (99%) du composé attendu sous forme d'huile marron clair qui cristallise en le maintenant à 4°C.

RMN-¹H (CDCl₃), δ : 1,58-1,67 (m, 4H), 2,58 (m, 2H), 2,83 (t, 2H, J = 6,8 Hz), 3,40-3,45 (q, 2H, J = 6,5 Hz), 3,51 (t, 2H, J = 6,8 Hz), 3,66 (s, 2H), 6,19 (s, 1H), 7,24-7,48 (m, 8H), 7,73-7,75 (m, 2H).

### 3ème étape

### N-[4-(benzyl-(2-{8-quinolyloxy}éthyl) amino)butyl]benzamide

### (V, Ar = 8-quinolyle, R=phényle, Y = -NHC(O)-)

On ajoute, par portions, de l'hydrure de sodium (0,63 g, 16 mmol), sous agitation à une solution refroidie (5°C) de 8-hydroxyquinoléine (2,3 g, 16 mmol) dans du diméthylformamide anhydre (80 ml). La solution est agitée 30 minutes à température ambiante (25°C), puis on ajoute goutte à goutte le N-[4-{benzyl (2-chloroéthyl) amino}butyl] benzamide (5 g, 14 mmol) en solution dans du diméthylformamide anhydre (80 ml). Le mélange réactionnel est maintenu à 60°C pendant 2 heures puis le solvant est évaporé sous pression réduite. Le résidu est repris dans du dichlorométhane (100 ml) puis lavé à l'eau et séché sur sulfate de magnésium. La filtration et l'évaporation du solvant sous pression réduite donnent une huile qui est purifiée par chromatographie flash sur gel de silice en utilisant un mélange acétate d'éthyle/heptane (2/1) comme éluant pour obtenir 6,1 g (95%) du composé souhaité, sous forme d'huile.

RMN-¹H (DMSO), δ : 1,50-1,70 (m, 4H), 2,54-2,61 (m, 2H), 2,93 (t, 2H, J = 5Hz), 3,24-3,30 (m, 2H), 3,73 (s, 2H), 4,25 (t, 2H, J = 5Hz), 7,00-7,60 (m, 10H), 7,70-7,90 (m, 2H), 8,30 (dd, 1H, J₁ = 2Hz, J₂ = 12Hz), 8,45 (t, 2H, J = 7Hz), 8,85 (dd, 1H, J₁ = 2Hz, J₂ = 4Hz).

### 4ème étape

### N-[4-({2-(8-quinolyloxy)éthyl}amino)butyl]benzamide

On ajoute, goutte à goutte, une solution de α-chloroéthylchloroformate (1,9 ml, 2,5 g, 17,5 mmol) dans du 1,2-dichloroéthane sec (10 ml) à une solution de N-[4-{benzyl [2-(8-quinoléinoxy)éthyl]amino}butyl] benzamide (4 g, 8,8 mmol) dans du 1,2-dichloroéthane sec (40 ml) sous atmosphère d'azote et à 5°C. Après une heure de chauffage à 60°C, le solvant est évaporé sous pression réduite, le résidu est repris par du méthanol (40 ml) et chauffé pendant une heure au reflux. Le solvant est concentré sous pression réduite puis le résidu repris par du dichlorométhane (50 ml) et de l'eau (50 ml). La phase aqueuse est récoltée et basifiée par une solution saturée d'hydrogénocarbonate de sodium puis le produit est extrait par 3 lavages par du dichlorométhane (30 ml). Après évaporation du solvant, le résidu est purifié par flash-chromatographie sur gel de silice dans un mélange dichlorométhane /méthanol (90/10) additionné de quelques gouttes de triéthylamine. On obtient 1,8 g (58%) du produit souhaité, sous forme d'huile incolore.

Le traitement d'une solution éthanolique de cette base libre (1,7 g) par une solution chaude d'acide fumarique (0,57 g) permet d'obtenir 1,5 g du fumarate du composé n°5, sous forme de cristaux blancs, pf = 144-149°C.

RMN-¹H (DMSO), δ : 1,60 (m, 4H), 3,00 (t, 2H, J = 7,4 Hz), 3,30 (m, 4H), 4,40 (t, 2H, J = 7,4Hz), 6,50 (s, 2H), 7,30 (dd, 1H, J₁ = 1,3Hz, J₂ = 6,23Hz), 7,40-7,60 (m, 6H), 7,85 (dd, 2H), 8,35 (dd, 1H), 8,55 (t, 1H), 8,90 (dd, 1H).

RMN-¹³C (DMSO), δ : 24,53 ; 26,55 ; 46,46 ; 47,64 ; 66,55 ; 112,73 ; 120,66 ; 121,98 ; 126,45 ; 127,09 ; 128,39 ; 129,10 ; 131,29 ; 134,64 ; 136,13 ; 139,82 ; 149,35 ; 153,88 ; 166,15 ; 167,59.

IR (Nujol), cm⁻¹ : 3302 (NH), 1709 (C = O), 1630 (C = O), 1577 (C = C), 1546 (C-N).

### EXEMPLE 2

### N-cyclohexyl-5-[(2-{8-quinolyloxy}éthyl)amino]pentanamide

(I, Ar = 8-quinolyle, R = cyclohexyle, Y = -C(O)NH- : composé n°11, Tableau 1)

### 1ère étape

### N-cyclohexyl-5-[benzyl-(2-hydroxyéthyl)amino]pentanamide

### (III, R = cyclohexyle, Y = -C(O)NH- )

On chauffe à 60°C pendant 15 minutes un mélange de N-benzyl éthanolamine (10,14 g, 67 mmol), de carbonate de potassium (18,53 g, 134 mmol) et d'iodure de potassium (0,5 g, 3 mmol) en suspension dans du diméthylformamide sec (100 ml). On ajoute ensuite goutte à goutte une solution de N-cyclohexyl-5-bromopentanamide (17,6 g, 67 mmol) dans du diméthylformamide (35 ml). Le milieu réactionnel est maintenu sous agitation à cette température pendant 2 heures. Les solides en suspension sont filtrés, puis de l'acétate d'éthyle (70 ml) et de l'eau (60 ml) sont ajoutés au milieu. La phase organique est récoltée, séchée sur sulfate de sodium. Après filtration et évaporation des solvants sous pression réduite, le produit obtenu, sous forme d'huile, est purifié par flash-chromatographie sur gel de silice dans un éluant dichlorométhane / méthanol /ammoniaque (90/10/1) pour obtenir 12,7 g (57%) du produit souhaité, sous forme d'huile incolore.
RMN-¹H (CDCl₃), δ : 1,00-1,90 (m, 14H), 2,05 (t, 2H, J = 6,4Hz), 2,40-2,65 (m, 4H), 3,50-3,85 (m, 5H), 5,30 (m, 1H), 7,30 (s, 5H).

### 2ème étape

### N-cyclohexyl-5-[benzyl-(2-chloroéthyl)amino]pentanamide

### (IV, R = cyclohexyle, Y = -C(O)NH-)

On ajoute goutte à goutte une solution de chlorure de méthanesulphonyle (3,8 g, 2,56 ml, 33 mmol) dans le dichlorométhane (30 ml), sous agitation, à une solution refroidie (5°C) de N-cyclohexyl-5-{benzyl (2-hydroxyéthyl)amino}pentanamide (9,15 g, 27,5 mmol) dans du dichlorométhane (30 ml) en présence de triéthylamine (3,62 g, 5 ml, 36 mmol). La réaction est maintenue à 20°C pendant une heure, puis le mélange réactionnel est lavé à l'eau glacée (50 ml) et séché sur sulfate de sodium. La filtration et l'évaporation du solvant sous pression réduite donnent 9,60 g (99%) du composé souhaité, sous forme d'huile jaune, qui est utilisé pour l'étape suivante sans autre purification.

### 3ème étape

### N-cyclohexyl-5-[benzyl-(2-{8-quinolyloxy}éthyl) amino] pentanamide

### (V, Ar = 8-quinolyle, R = cyclohexyle, Y = -C(O)NH- )

On ajoute, par portions, de d'hydrure de sodium (0,23 g, 5,7 mmol), sous agitation, à une solution refroidie (5°C) de 8-hydroxyquinoléine (0,83 g, 5,7 mmol) dans du diméthylformamide anhydre (10 ml). La solution est agitée 15 minutes à 20°C puis le N-cyclohexyl-4-{benzyl(2-chloroéthyl)amino}butanamide (2 g, 5,7 mmol) en solution dans du diméthylformamide anhydre (10 ml) est ajouté goutte à goutte. Le mélange réactionnel est maintenu à 50°C pendant 3 heures puis de l'acétate d'éthyle (10 ml) et de l'eau (10 ml) sont ajoutés au milieu. La phase organique est collectée puis lavée par une solution de soude 1N, et séchée sur sulfate de sodium. Après filtration et évaporation, le produit attendu est purifié par chromatographie flash sur gel de silice dans un mélange dichlorométhane/méthanol (95/5). On obtient 1,1 g (41%) de produit, sous forme d'huile incolore.

### 4ème étape

### N-cyclohexyl-5-[(2-{8-quinolyloxy}éthyl)amino)pentanamide

On ajoute, goutte à goutte, une solution d'α-chloroéthylchloroformate (0,67 g, 0,51 ml, 4,7 mmol) dans du 1,2-dichloroéthane sec (3 ml) à une solution de N-cyclohexyl-5-{benzyl[2-(8-quinoléinoxy)éthyl]amino}pentanamide(1,08 g, 2,35 mmol) dans du 1,2-dichloroéthane sec (10 ml) sous atmostphère d'azote et à 5°C. Après deux heures de chauffage à 40°C, le solvant est évaporé sous pression réduite ; le résidu est repris par du méthanol (30 ml) et chauffé pendant 30 minutes à reflux. Le solvant est concentré sous pression réduite puis le résidu est repris par du dichlorométhane (30 ml) et lavé par une solution de soude 2N. La phase organique est collectée puis séchée sur sulfate de sodium. Après filtration et évaporation du solvant, le produit est purifié par flash-chromatographie sur gel de silice dans un mélange dichlorométhane/méthanol/ammoniaque (80/20/2). Le produit est obtenu sous forme d'huile incolore (0,24 g, 28%).

Le traitement d'une solution de cette base libre (0,2 g) dans l'acétone par une solution acétonique d'acide fumarique permet d'obtenir du fumarate du composé n° 11 (0,12 g) sous forme de cristaux beiges, pf = 113-119°C.

RMN-¹H (DMSO), δ : 1,00-1,30 (m, 5H), 1,50-1,75 (m, 10H), 2,10 (t, 2H, J = 6,4Hz), 3,00 (t, 2H, J = 7,2Hz), 3,30 (t, 2H, J = 5,2 Hz), 3,50 (m, 1H), 4,40 (t, 2H, J = 5,2Hz), 6,50 (s, 2H), 7,30 (d, 1H, J = 7,2Hz), 7,55 (m, 3H), 7,70 (d, 1H, J = 7,8Hz), 8,35 (dd, 1H, J₁ = 1,3Hz, J₂ = 8,2Hz), 8,85 (dd, 1H, J₁ = 1,4Hz, J₂ = 4Hz).

RMN-¹³C (DMSO), δ : 22,89 ; 24,79 ; 25,42 ; 26,32 ; 32,71 ; 35,12 ; 46,37 ; 47,47 ; 47,64 ; 66,44 ; 111,36 ; 120,87 ; 122,16 ; 127,05 ; 129,32 ; 135,11 ; 136,28; 140,00 ; 149,36 ; 153,99 ; 167,76 ; 170,90.

IR (Nujol), cm⁻¹ : 3308 (N-H), 1704 (C = O), 1641 (C = O), 1540 (C-N).

Les procédés décrits ci-dessus donnent un composé de l'invention sous forme d'une base-libre ou d'un sel d'addition avec un acide. Si le composé de l'invention est obtenu sous la forme d'un sel d'addition avec un acide, la base libre peut être obtenue en basifiant une solution du sel d'addition avec une base. A l'inverse, si le produit du procédé est une base libre, le sel d'addition avec un acide, en particulier un sel d'addition avec un acide pharmaceutiquement acceptable, peut être obtenu en dissolvant la base libre dans un solvant organique approprié et en traitant la solution avec un acide, selon les procédures conventionnelles de préparation des sels d'addition avec un acide à partir des bases libres.

Des exemples de sels d'addition avec un acide sont ceux dérivés d'acides inorganiques tels que les acides sulfurique, chlorhydrique, bromhydrique ou phosphorique, ou d'acides organiques tels que les acides tartrique, fumarique, maléique, citrique, caprylique, benzoïque, méthanesulphonique, p-toluènesulphonique, benzène-sulphonique, succinique ou acétique.

Pour les composés de l'invention contenant un centre asymétrique, les mélanges racémiques et les isomères optiquement actifs individuels sont également considérés comme faisant partie de la portée de l'invention.

Le Tableau 1 ci-dessous montre les principaux composés préparés selon les procédures ci-dessus et qui illustrent l'invention sans en limiter la portée. Les composés Nos. 5 et 11 correspondent respectivement aux produits des exemples 1 et 2 décrits ci-dessus. Les autres produits ont été préparés en utilisant le même procédé.

**Tableau 1**

| Composé No. | Ar | Y | R | Sel | pf(°C) |
|---|---|---|---|---|---|
| 1 | 8-quinolyle | -NHC(O)- | *tert*butyle | fumarate | 141-143,5 |
| 2 | 8-quinolyle | -NHC(O)- | néopentyle | fumarate | 110,5-111 |
| 3 | 8-quinolyle | -NHC(O)- | cyclohexyle | fumarate | 125-127,5 |
| 4 | 8-quinolyle | -NHC(O)- | 1-méthyl cyclohexyle | fumarate | 113,5-114,5 |
| 5 | 8-quinolyle | -NHC(O)- | phényle | fumarate | 146-149 |
| 6 | 5-CI-8-quinolyle | -NHC(O)- | néopentyle | 1,5-fumarate | 122,5-125 |
| 7 | 2-Me-8-quinolyle | -NHC(O)- | néopentyle | fumarate | 95-97 |
| 8 | 5-quinolyle | -NHC(O)- | phényle | hémifumarate | 168-170 |
| 9 | 5-quinoxalyle | -NHC(O)- | phényle | dichlorhydrate | 178-180 |
| 10 | 2-OH-8-quinolyle | -NHC(O) | phényle | hémifumarate | 177-180 |
| 11 | 8-quinolyle | -C(O)NH- | cyclohexyle | fumarate | 113-119 |
| 12 | 8-quinolyle | -C(O)NH- | néopentyle | fumarate | 109,5-111 |
| 13 | 5-quinoxalyle | -C(O)NH- | néopentyle | 0,75-fumarate | 131-133 |

En utilisant le procédé indiqué ci-dessus, on peut également préparer les produits suivants, qui font également partie de l'invention :

| **Composé** | **Ar** | **Y** | **R** |
|---|---|---|---|
| A | 8-quinolyle | -C(O)NH- | phényle |
| B | 5-quinoxalyle | -C(O)NH- | 4-chlorophényle |
| C | 5-F-8-quinolyle | -C(O)NH- | cyclohexyle |
| D | 5-isoquinolyle | -C(O)NH- | méthyle |
| E | 8-Me-7-isoquinolyle | -C(O)NH- | cyclopentyle |
| F | 8-quinolyle | -C(O)NH- | 4-méthylcyclohexyle |
| G | 8-Me-7-isoquinolyle | -C(O)NH- | cycloheptyle |
| H | 5-quinoxalyle | -NHC(O)- | 3-méthoxyphényle |
| I | 5-isoquinolyle | -NHC(O)- | cyclopentyle |
| J | 5-isoquinolyle | -NHC(O)- | propyle |

### Etude pharmacologique des produits de l'invention

### Affinité des composés de l'invention pour le récepteur 5-HT_{1A}

L'affinité des composés pour les récepteurs 5-HT_{1A} sérotonergiques est déterminée en mesurant l'inhibition de [3H]8-hydroxy-2(di-n-propylamino)tétralin([3H]8-OH-DPAT) lié au cortex cérébral du rat, selon la méthode de Peroutka et ses collaborateurs [(J. Neurochem., 47, 529 (1986)].

Des cortex cérébraux de rats Sprague Dawley mâles sont homogénéisés dans du Tris-HCl 50 mM, pH = 7,4 et centrifugés à 40 000 g pendant 10 min à 4° C. Des pastilles sont remises en suspension dans le même tampon et incubées pendant 10 min à 37° C, et les homogénats sont de nouveau centrifugés à 40 000 g pendant 10 min à 4° C.

Des essais d'inhibition compétitive de liaison de [3H]8-OH-DPAT sont réalisés trois fois avec des compétiteurs non marqués, avec des concentrations comprises entre 100 pM et 100 µM. Des membranes de cortex cérébraux de rats (10 mg en poids humide/ ml) sont incubées avec du [3H]8-OH-DPAT (1 nM) pendant 30 min à 25° C dans du Tris-HCl 50 mM, pH = 7,4 contenant 4 mM de CaCl₂, 10 mM de pargyline et 0,1 % d'acide ascorbique.

Le [3H]8-OH-DPAT lié est séparé du [3H]8-OH-DPAT libre par filtration immédiate au moyen de filtres en fibre de verre Whatman GF/B, en utilisant un récupérateur de cellules Brandel. Les filtres sont lavés trois fois avec le même tampon à 0-4° C et leur radioactivité est mesurée au moyen d'un spectromètre à scintillation liquide.

La liaison spécifique est obtenue en soustrayant la liaison déterminée en présence de 1 µM de 8-OH-DPAT de la liaison totale. Les caractéristiques de la liaison sont analysées par analyse itérative de la régression non linéaire par ordinateur, en utilisant le programme Ligand [Munson and Rodbard, Anal. Biochem., 107, 220 (1980)].

Les résultats pour des composés représentatifs de l'invention sont donnés dans le Tableau 2 ci-après.

**Tableau 2**

| **Composé No.** | **Ki(nM)** |
|---|---|
| 1 | 0,081 |
| 2 | 0,12 |
| 3 | 0,15 |
| 4 | 0,11 |
| 5 | 0,055 |
| 6 | 0,98 |
| 7 | 0,35 |
| 9 | 0,75 |
| 11 | 0,072 |
| 12 | 0,09 |
| 13 | 0,48 |

## Revendications

1. Les composés de formule générale I: 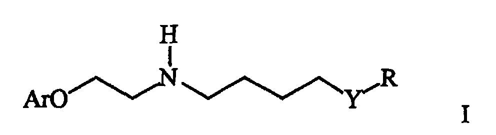 dans laquelle
Ar représente un radical hétéroaryle bicyclique contenant un ou deux atomes d'azote, éventuellement substitué par un ou plusieurs substituants choisis parmi les radicaux hydroxy, nitro, halo, alkyle inférieur, alkoxy inférieur, alkyle inférieur carbonyle;
Y représente le radical -C(O)NH- ou -NHC(O)- ;
R représente un radical alkyle inférieur ; un cycloalkyle monocyclique saturé ayant de 3 à 7 atomes de carbone et éventuellement substitué par un ou plusieurs alkyle(s) inférieur(s) ; ou un phényle éventuellement substitué par un ou plusieurs substituants choisis parmi les radicaux hydroxy, cyano, halo, trifluorométhyle, alkyle inférieur, alkoxy inférieur ;
ainsi que les sels de ces produits, étant entendu que le terme "inférieur" lorsqu'il se réfère aux termes "alkyle" ou "alkoxy", signifie de 1 à 6 atomes de carbone.

2. Les composés de formule générale I telle que définie à la revendication 1, **caractérisée en ce que** Ar représente le radical quinolyle ou quinoxalinyle, et le ou les substituant(s) que peut porter le radical que représente Ar sont choisis parmi les radicaux hydroxy, chloro, méthyle.

3. Les composés de formule générale I telle que définie à l'une des revendications 1 à 2 et répondant aux formules suivantes :
- N-[4-({2-(8-quinolyloxy)éthyl}amino)butyl]*tert*butanamide,
- N-[4-({2-(8-quinolyloxy)éthyl} amino)butyl]néopentanamide,
- N-[4-({2-(8-quinolyloxy)éthyl}amino)butyl]cyclohexanamide,
- 4-méthyl-N-[4-( {2-(8-quinolyloxy)éthyl} amino)butyl]cyclohexanamide,
- N-[4-({2-(8-quinolyloxy)éthyl}amino)butyl]benzamide,
- N-[4-({2-(8-(5-chloro)quinolyloxy)éthyl}amino)butyl]néopentanamide,
- N-[4-({2-(8-(2-méthyl)quinolyloxy)éthyl}amino)butyl]néopentanamide,
- N-[4-({2-(5-quinolyloxy)éthyl}amino)butyl]benzamide,
- N-[4-({2-(5-quinoxalyloxy)éthyl}amino)butyl]benzamide,
- N-[4-({2-(8-(2-hydroxy)quinolyloxy)éthyl} amino)butyl]benzamide,
- N-cyclohexyl-5-[(2-{8-quinolyloxy}éthyl)amino]pentanamide,
- N-néopentyl-5-[(2- {8-quinolyloxy}éthyl)amino]pentanamide,
- N-néopentyl-5-[(2- {5-quinoxalyloxy} éthyl)amino]pentanamide,
ainsi que les sels de ces composés avec les acides minéraux ou organiques.

4. Procédé de préparation des produits de formule générale I telle que définie à la revendication 1, **caractérisé en ce que**
l'on fait réagir un produit de formule II 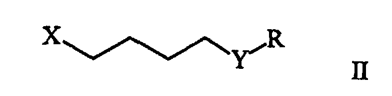 dans laquelle Y et R ont la signification indiquée ci-dessus et X représente un halogène ou un pseudo halogène, avec la N-benzyléthanolamine de formule 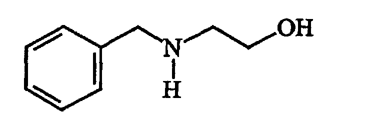 pour obtenir un produit de formule III 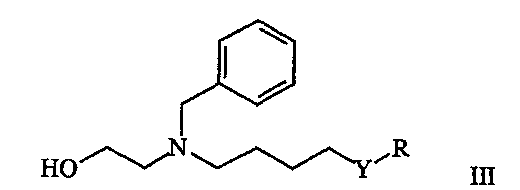 que l'on transforme en produit de formule IV 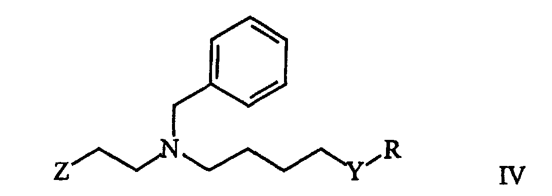 dans laquelle Z représente un halogène ou un pseudo halogène, produit de formule IV que l'on transforme en produit de formule V 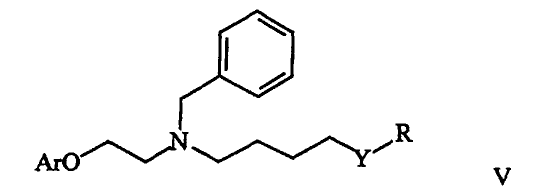 dans laquelle Ar a la signification indiquée ci-dessus, produit de formule V dont la fonction benzyle est clivée pour obtenir le produit de formule I, et produit de formule I que l'on peut transformer en sels d'acide par action de l'acide correspondant.

5. A titre de médicaments, les produits de formule I telle que définie à la revendication 1, ainsi que les sels d'addition avec les acides minéraux ou organiques pharmaceutiquement acceptables desdits produits de formule I.

6. A titre de médicaments, les produits de formule I telle que définie à l'une des revendications 2 à 3, ainsi que les sels d'addition avec les acides minéraux ou organiques pharmaceutiquement acceptables desdits produits de formule I.

7. Compositions pharmaceutiques contenant, à titre de principe actif, l'un au moins des médicaments tels que définis à l'une des revendications 5 ou 6.

8. Utilisation des produits de formule I telle que définie à l'une quelconque des revendications 1 à 3 pour la préparation de médicaments antiémétiques.

9. Utilisation des produits de formule I telle que définie à l'une quelconque des revendications 1 à 3 pour la préparation de médicaments destinés à réduire la sécrétion gastrique.

10. Utilisation des produits de formule I telle que définie à l'une quelconque des revendications 1 à 3 pour la préparation de médicaments destinés à accélérer la vidange gastrique.

11. Utilisation des produits de formule I telle que définie à l'une quelconque des revendications 1 à 3 pour la préparation de médicaments destinés à modifier le transit intestinal.

12. Utilisation des produits de formule I telle que définie à l'une quelconque des revendications 1 à 3 pour la préparation de médicaments destinés à traiter l'anxiété, la dépression, les troubles du sommeil.

13. Utilisation des produits de formule I telle que définie à l'une quelconque des revendications 1 à 3 pour la préparation de médicaments destinés à traiter les maladies cardiovasculaires, notamment l'hypertension.

14. A titre de produits industriels, les composés de formule V telle que définie à la revendication 4.

## Claims

1. Compounds of general formula I 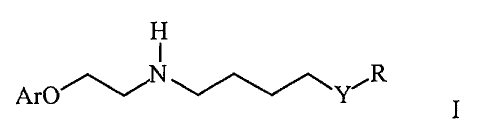 in which
Ar represents a bicyclic heteroaryl radical containing one or two nitrogen atoms, optionally substituted with one or more substituents selected from hydroxy, nitro, halo, lower alkyl, lower alkoxy, lower alkyl carbonyl radicals ;
Y represents the -C(O)NH- or -NHC(O)- radical;
R represents a lower alkyl radical ; a saturated monocyclic cycloalkyl containing from 3 to 7 carbon atoms and optionally substituted with one or more lower alkyl radical(s) ; or a phenyl optionally substituted with one or more substituents selected from hydroxy, cyano, halo, trifluoromethyl, lower alkyl, lower alkoxy radicals;
and the salts of these products, it being understood that the word « lower », when it refers to the words « alkyl » or « alkoxy », means from 1 to 6 carbon atoms.

2. Compounds of general formula I as defined in claim 1, **characterised in that** Ar represents the quinolyl or quinoxalinyl radical, and that any substituent(s) carried by the radical represented by Ar are selected from hydroxy, chloro and methyl radicals.

3. Compounds of general formula I as defined in any of claims 1 to 2, and corresponding to the following formulae :
- N-[4-({2-(8-quinolyloxy)ethyl}amino)butyl]tertbutanamide,
- N-[4-({2-(8-quinolyloxy)ethyl)amino)butyl]neopentanamide,
- N-[4-({2-(8-quinolyloxy)ethyl}amino)butyl]cyclohexanamide,
- 4-methyl-N-[4-({2-(8-quinolyloxy)ethyl}amino)butyl]cyclohexanamide,
- N-[4-({2-(8-quinolyloxy)ethyl}amino)butyl]benzamide,
- N-[4-({2-(8-(5-chloro)quinolyloxy)ethyl}amino)butyl]neopentanamide,
- N-[4-({2-(8-(2-methyl)quinolyloxy)ethyl}amino)butyl]neopentanamide,
- N-[4-({2-(5-quinolyloxy)ethyl}amino)butyl]benzamide,
- N-[4-({2-(5-quinoxalyloxy)ethyl}amino)butyl]benzamide,
- N-[4-({2-(8-(2-hydroxy)quinolyloxy)ethyl}amino)butyl]benzamide,
- N-cyclohexyl-5-[(2- {8-quinolyloxy}ethyl)amino]pentanamide,
- N-neopentyl-5-[(2-{8-quinolyloxy}ethyl)amino]pentanamide,
- N-neopentyl-5-[(2-{5-quinoxalyloxy}ethyl)amino]pentanamide,
as well as the salts of these compounds with mineral or organic acids.

4. A process for the preparation of products of general formula I as defined in claim 1, **characterised in that** a product of formula II, 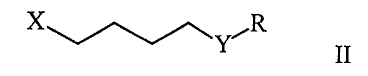 in which Y and R have the meaning indicated above and X represents a halogen or a pseudo-halogen, is reacted with N-benzylethanolamine of formula 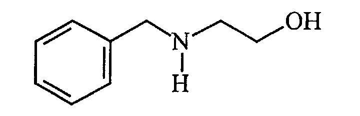 in order to obtain a product of formula III 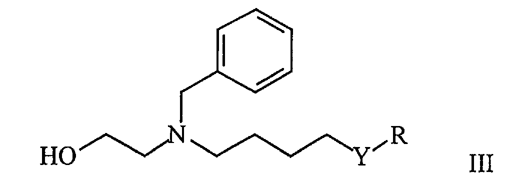 which is converted into a product of formula IV 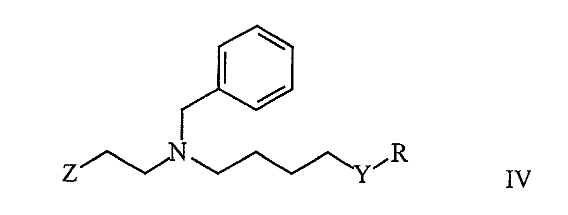 in which Z represents a halogen or a pseudo-halogen, product of formula IV which is converted into a product of formula V 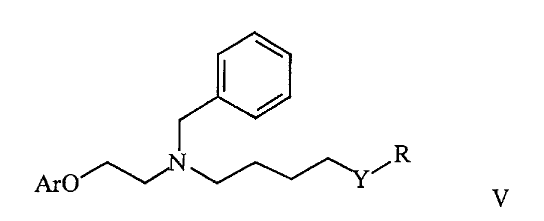 in which Ar has the meaning indicated above, product of formula V whose benzyl function is cleaved in order to obtain a product of formula I, and product of formula I which can be converted into acid salts by the action of the corresponding acid.

5. As medicaments, products of formula I as defined in claim 1, and addition salts with pharmaceutically acceptable mineral or organic acids of said products of formula I.

6. As medicaments, products of formula I as defined in any of claims 2 to 3, as well as addition salts with pharmaceutically acceptable mineral or organic acids of said products of formula I.

7. Pharmaceutical compositions containing, as active ingredient, at least one medicament as defined in any of claims 5 to 6.

8. Use of products of formula I as defined in any of claims 1 to 3, for the preparation of antiemetic drugs.

9. Use of products of formula I as defined in any of claims 1 to 3, for the preparation of drugs intended to reduce gastric secretion.

10. Use of products of formula I as defined in any of claims 1 to 3, for the preparation of drugs intended to accelerate gastric emptying.

11. Use of products of formula I as defined in any of claims 1 to 3, for the preparation of drugs intended to modify intestinal transit.

12. Use of products of formula I as defined in any of claims 1 to 3, for the preparation of drugs intended to treat anxiety, depression, sleep disorders.

13. Use of products of formula I as defined in any of claims 1 to 3, for the preparation of drugs intended to treat cardiovascular diseases, notably hypertension.

14. As industrial products, compounds of formula V as defined in claim 4.

## Patentansprüche

1. Verbindungen der allgemeinen Formel I: 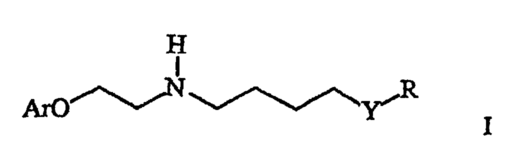 in der
Ar einen bicyklischen Heteroarylrest darstellt, der ein oder zwei Stickstoffatome enthält und ggf. durch einen oder mehrere Substituenten substituiert ist, die aus den Resten Hydroxy, Nitro, Halogen, Niederalkyl, Niederalkoxy, Niederalkylcarbonyl ausgewählt sind;
Y den Rest -C(O)NH- oder -NHC(O)- darstellt;
R einen Niederalkylrest, ein gesättigtes monocyklisches Cycloalkyl mit 3 bis 7 Kohlenstoffatomen, das ggf. durch ein oder mehrere Niederalkyl(e) substituiert ist, oder ein Phenyl darstellt, das ggf. durch einen oder mehrere Substituenten substituiert ist, die aus den Resten Hydroxy, Cyan, Halogen, Trifluormethyl, Niederalkyl, Niederalkoxy ausgewählt sind;
sowie die Salze dieser Produkte, wobei der Begriff "nieder", wenn er sich auf die Begriffe "Alkyl" oder "Alkoxy" bezieht, 1 bis 6 Kohlenstoffatome bedeutet.

2. Verbindungen der allgemeinen Formel I gemäß Anspruch 1, **dadurch gekennzeichnet, dass** Ar einen Chinolyl- oder Chinoxalinylrest darstellt und der oder die Substituent(en), die der Rest, den Ar darstellt, tragen kann, aus den Resten Hydroxy, Chlor, Methyl ausgewählt sind.

3. Verbindungen der allgemeinen Formel I gemäß einem der Ansprüche 1 bis 2, die den folgenden Formeln entsprechen:
- N-[4-({2-(8-Chinolyloxy)ethyl}amino)butyl] *tert*.butanamid,
- N-[4-({2-(8-Chinolyloxy)ethyl}amino)butyl]neopentanamid,
- N-[4-({2-(8-Chinolyloxy)ethyl}amino)butyl]cyclohexanamid,
- 4-Methyl-N-[4-({2-(8-chinolyloxy)ethyl}amino)butyl]-cyclohexanamid,
- N-[4-({2-(8-Chinolyloxy)ethyl}amino)butyl]benzamid,
- N-[4-({2-(8-(5-Chlor)chinolyloxy)ethyl}amino)butyl]neopentanamid,
- N- [4-({2-(8-(2-Methyl)chinolyloxy)ethyl}amino)butyl]neopentanamid,
- N-[4-({2-(5-Chinolyloxy)ethyl}amino)butyl]benzamid,
- N-[4-({2-(5-Chinoxalyloxy)ethyl}amino)butyl]benzamid,
- N- [4- ({2-(8-(2-Hydroxy)chinolyloxy)ethyl}amino)butyl] benzamid,
- N-Cyclohexyl-5-[(2-{8-chinolyloxy}ethyl)amino]pentanamid,
- N-Neopentyl-5-[(2-{8-chinolyloxy}ethyl)amino]pentanamid,
- N-Neopentyl-5-[(2-{5-chinoxalyloxy}ethyl)amino]pentanamid,
sowie die Salze dieser Verbindungen mit den anorganischen oder organischen Säuren.

4. Verfahren zur Herstellung der Produkte der allgemeinen Formel I gemäß Anspruch 1, **dadurch gekennzeichnet, dass** man ein Produkt der Formel II 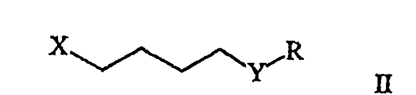 in der Y und R die oben angegebene Bedeutung haben und X ein Halogen oder ein Pseudohalogen darstellt,
mit N-Benzylethanolamin der Formel 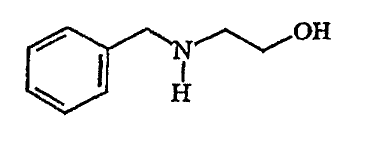 reagieren lässt, um ein Produkt der Formel III zu erhalten, 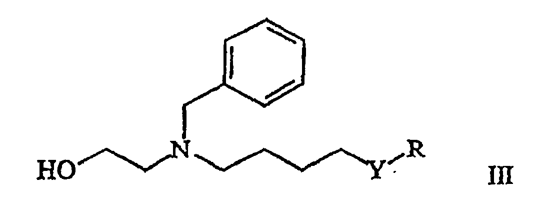 das man in ein Produkt der Formel IV umwandelt 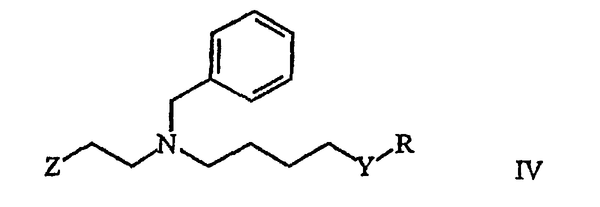 in der Z ein Halogen oder ein Pseudohalogen darstellt, wobei man das Produkt der Formel IV in ein Produkt der Formel V umwandelt, 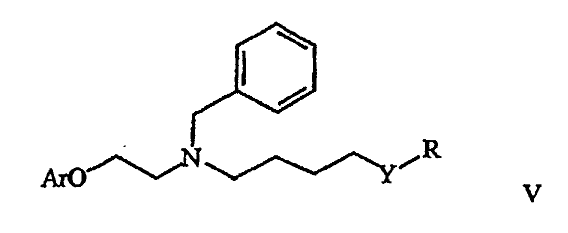 in der Ar die oben angegebene Bedeutung hat, wobei die Benzylfunktion des Produkts der Formel V aufgespalten wird, um das Produkt der Formel I zu erhalten, das man durch Einwirkung der entsprechenden Säure in Säuresalze umwandeln kann.

5. Produkte der Formel I gemäß Anspruch 1 sowie die Additionssalze dieser Produkte der Formel I mit den pharmazeutisch verträglichen anorganischen oder organischen Säuren in Form von Medikamenten.

6. Produkte der Formel I gemäß einem der Ansprüche 2 bis 3 sowie die Additionssalze dieser Produkte der Formel I mit den pharmazeutisch verträglichen anorganischen oder organischen Säuren in Form von Medikamenten.

7. Pharmazeutische Zusammensetzungen, die als Wirkstoff mindestens eines der Medikamente gemäß einem der Ansprüche 5 oder 6 enthalten.

8. Verwendung der Produkte der Formel I gemäß einem der Ansprüche 1 bis 3 für die Herstellung von antiemetischen Medikamenten.

9. Verwendung der Produkte der Formel I gemäß einem der Ansprüche 1 bis 3 für die Herstellung von Medikamenten zur Reduzierung der Magensekretion.

10. Verwendung der Produkte der Formel I gemäß einem der Ansprüche 1 bis 3 für die Herstellung von Medikamenten zur Beschleunigung der Magenentleerung.

11. Verwendung der Produkte der Formel I gemäß einem der Ansprüche 1 bis 3 für die Herstellung von Medikamenten zur Modifizierung des Darmdurchgangs.

12. Verwendung der Produkte der Formel I gemäß einem der Ansprüche 1 bis 3 für die Herstellung von Medikamenten zur Behandlung von Angstzuständen, Depressionen, Schlafstörungen.

13. Verwendung der Produkte der Formel I gemäß einem der Ansprüche 1 bis 3 für die Herstellung von Medikamenten zur Behandlung von kardiovaskulären Erkrankungen, insbesondere Bluthochdruck.

14. Verbindungen der Formel V gemäß Anspruch 4 in Form von Industrieprodukten.
